(19) **Europäisches Patentamt European Patent Office Office européen des brevets**

(11) **EP 1 198 632 B1**

(12) **EUROPEAN PATENT SPECIFICATION**

(45) Date of publication and mention of the grant of the patent:
**01.02.2012 Bulletin 2012/05**

(51) Int Cl.:
**D04H 13/00** (2006.01)   **B29C 55/14** (2006.01)
**B29C 55/06** (2006.01)   **D04H 1/44** (2006.01)

(21) Application number: **00950887.0**

(22) Date of filing: **27.07.2000**

(86) International application number:
**PCT/US2000/020756**

(87) International publication number:
**WO 2001/009424 (08.02.2001 Gazette 2001/06)**

(54) **CD EXTENSIBLE CLOTH-LIKE NONWOVEN FOR FACING AND LINER**

FASERVLIES FÜR DECKSCHICHT UND EINLAGE MIT DEHNBARKEIT IN DER QUERRICHTUNG

NON TISSE DE TYPE TISSU EXTENSIBLE DANS LE SENS DE LA TRAME UTILISE COMME PAREMENTURE ET DOUBLURE

(84) Designated Contracting States:
**DE FR GB**

(30) Priority: **28.07.1999 US 363124**

(43) Date of publication of application:
**24.04.2002 Bulletin 2002/17**

(73) Proprietor: **KIMBERLY-CLARK WORLDWIDE, INC. Neenah, WI 54956 (US)**

(72) Inventors:
 • **YING, Sandy, Chi-Ching Roswell, GA 30076 (US)**
 • **MORMAN, Michael, Tod Alpharetta, GA 30022 (US)**
 • **SHAWVER, Susan, Elaine Roswell, GA 30076 (US)**
 • **ESTEY, Paul, Windsor Cumming, GA 30041 (US)**
 • **SHULTZ, Jay, Sheldon Roswell, GA 30041 (US)**
 • **UITENBROEK, Duane, Girard Little Chute, WI 54140 (US)**

(74) Representative: **Davies, Christopher Robert et al Dehns St Bride's House 10 Salisbury Square London EC4Y 8JD (GB)**

(56) References cited:
**WO-A-98/48091     WO-A-99/37840
US-A- 4 116 892     US-A- 4 965 122
US-A- 5 226 992     US-A- 5 914 084**

**EP 1 198 632 B1**

**Description**

BACKGROUND OF THE INVENTION

Field of the Invention

This invention relates to elasticized materials and a method for making same. More particularly, this invention relates to cloth-like, cross-machine direction extensible, nonwoven webs including nonwoven webs which, in addition to being CD extensible, are also full width manufacturable, or necked nonwoven webs which are easily CD extensible much beyond their original manufactured width, and methods for producing such nonwoven webs. This invention also relates to nonwoven webs which, when attached to an elastic filament or nonwoven material, such as a film, foam or meltblown, constitute stretchable laminates. Such nonwoven webs and laminates are suitable for use as facings, outer covers and liners in a variety of articles including personal care articles such as diapers, incontinence garments, feminine care products such as sanitary pads and napkins, and in other types of articles such as wipes, garments including face masks, surgical gowns and the like, tissues, bandages, dressings and the like.

Description of Prior Art

Nonwoven webs formed by nonwoven extrusion processes, such as, for example, meltblowing and spunbonding processes, may be manufactured into products or components of products so inexpensively that the products may be disposed of after only one or a few uses. Such products include diapers, training pants, incontinence garments, wipes and feminine care products. However, nonwoven webs formed from nonelastic polymers normally lack elasticity, thereby restricting use of these nonwoven web materials in applications where elasticity is desirable or necessary, such as in disposable diapers, incontinence garments and sanitary pads and napkins.

Some of the problems in this area are the provision of elastic materials which are resilient and flexible while still feeling pleasant to the touch and which do not feel plastic or rubbery. The aesthetic properties of elastic materials can be improved by forming a laminate of an elastic material with one or more nonelastic materials on the outer face or surface which provide better tactile or hand properties. Composite materials of elastic and nonelastic material have been made by bonding the nonelastic material to the elastic material in a manner that allows the entire composite material to stretch or elongate and recover. In one such composite material, a nonelastic material is joined to the elastic material while the elastic material is in a stretched condition so that when the elastic material is relaxed, the nonelastic material gathers between the points at which it is bonded to the elastic material. As a result, the composite elastic material is stretchable to the extent that the nonelastic material gathered between the bond points allows the elastic material to elongate. Such a composite is taught, for example, by U.S. Patent 4,720,415 to Vander Wielen et aL

Currently, in the manufacture of some stretchable, cloth-like materials for personal care articles, the nonwoven facings are necked and then laminated to produce a cross-machine direction stretchable material, However, as a result of the necking of the nonwoven web facings, full width use of the base machine is lost, resulting in a loss of base machine productivity as measured in square yards per hour. Accordingly, in order to take full advantage of the base machine capacity, it is desirable to be able to use the entire width of the base machine for processing of the material while producing a nonwoven web and laminate which are cross-machine direction stretchable.

As indicated hereinabove, necking of nonwoven web materials to impart extensible properties thereto is known in the art. See, for example, U.S. Patent 4,965,122 to Morman. However, one problem with necked material is that it can only be extended to approximately its original manufactured width. As a material can only be necked so far before it breaks, the amount of stretch that can be imparted to the material is limited. A prior art method having the features of the preamble of claim 1, is disclosed in US-5914084.

SUMMARY OF THE INVENTION

Accordingly, it is one object of this invention to provide a method for producing cross-machine direction extensible nonwoven web materials which utilizes the full width of the base machine.

It is another object of this invention to provide a nonwoven web material which is extensible in the cross-machine direction to greater than the original machine width.

It is yet another object of this invention to provide a nonwoven web material which is highly stretchable.

According to the present invention, there is provided a method as claimed in claim 1.

These and other objects of this invention are addressed by a method for producing a nonwoven web material having cross-direction extensibility in which a nonwoven web is conveyed through means for elongating the nonwoven web in a cross-machine direction. The elongated nonwoven web is then necked, forming a cross-machine direction extensible nonwoven web material. In accordance with the method of this invention, the nonwoven web is necked to less than its original width resulting in a very high cross-direction stretch nonwoven, higher than just necking the original web. Indeed, the nonwoven web produced in accordance with this embodiment may be stretched up to about seven times its necked width. To lock in the necked width and give the web some retractive force from extension, the extensible nonwoven web material is heat treated. To form a cross-direction stretchable laminate in accordance with one embodiment, the extensible nonwoven web is attached to an elastic nonwoven material, for example, a film.

BRIEF DESCRIPTION OF THE DRAWINGS

These and other objects and features of this invention will be better understood from the following detailed description taken in conjunction with the drawings, wherein:

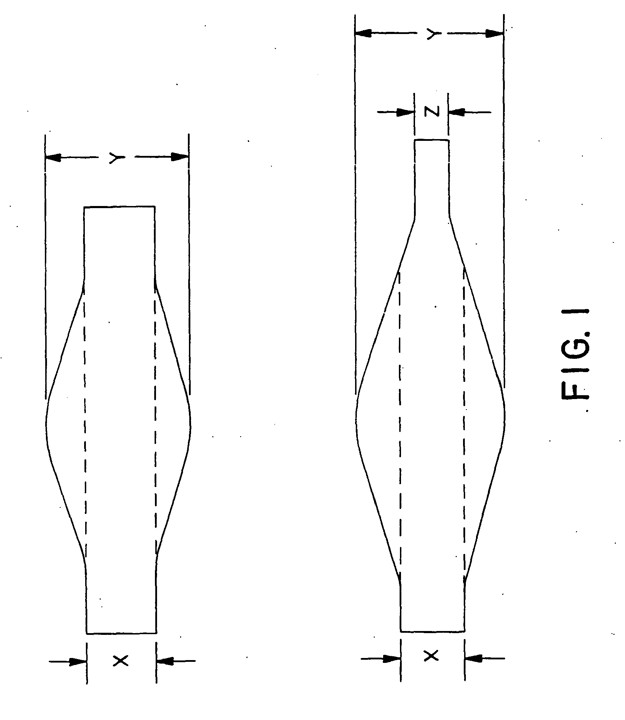

Fig. 1 is a diagram showing a top view of a nonwoven web material as it is processed through the elongation and necking steps of this invention.

DESCRIPTION OF PREFERRED EMBODIMENTS

Definitions

As used herein, the term "recover" refers to an immediate contraction of a stretched material upon termination of a biasing force following stretching of the material by application of the biasing force. For example, if a material having a relaxed, unbiased length of 1 in. (2.5 cm) is elongated fifty percent by stretching to a length of 1-½ in. (3.75 cm), the material would be elongated 50 percent and would have a stretched length that is 150 percent of its relaxed length. If this exemplary stretched material contracted, that is recovered to a length of 1·1/10 inches (1 inch=2·54 cm) after release of the biasing and stretching force, the material would have recovered 80 percent of its ½ inch (1.25 cm) elongation. Percent recovery may be expressed as [(maximum stretch length - final sample length)/(maximum stretch length - initial sample length)] x 100.

As used herein, the term "nonwoven web" means a web that has a structure of individual fibers or threads which are interlaid, but not in an identifiable repeating manner. Nonwoven webs have been, in the past, formed by a variety of processes such as, for example, meltblowing processes, spunbonding processes, coforming processes and bonded carded web processes.

As used herein, the term "necked material" refers to any material which has been constricted in at least one dimension by processes such as, for example, drawing or gathering.

As used herein, the term "neckable material" means any material which can be necked.

As used herein, the term "stretchable" refers to necked materials arid laminates of necked materials which have extensibility in a direction substantially parallel to the direction of necking in the range of about 100% of its necked width.

As used herein, the term "highly stretchable" or "highly stretchable materials" refers to necked materials and laminates of necked materials which have extensibility in a direction substantially parallel to the direction of necking in the range of about 3 to about 7 times its necked width.

As used herein, the term "percent stretch" refers to the ratio determined by measuring the increase in the stretched dimension and dividing that value by the original dimension, i.e. (increase in stretched dimension/original dimension) x 100.

As used herein, the term "machine direction" or "MD" means the length of a fabric in the direction in which it is produced. The term "cross machine direction" or "CD" means the width of fabric, that is a direction generally perpendicular to the MD.

As used herein, the term "spunbond fibers" refers to small diameter fibers which are formed by extruding molten thermoplastic material as filaments from a plurality of fine, usually circular capillaries of a spinneret, with the diameter of the extruded filaments then being rapidly reduced as by, for example, in U.S. Patent 4,340,563 to Appel et al., U.S. Patent 3,692,618 to Dorschner et al., U.S. Patent 3,802,817 to Matsuki et al., U.S. Patent 3,338,992 and 3,341,394 to Kinney, U.S. Patent 3,502,763 to Hartmann, and U.S. Patent 3,542,615 to Dobo et al. Spunbond fibers are generally not tacky when they are deposited onto a collecting surface. Spunbond fibers are generally continuous and have average diameters (from a sample of at least 10 fibers) larger than 7 microns, more particularly, between about 10 and 30 microns. The fibers may also halve shapes such as those described in U.S. Patent 5,277,976 to Hogle et al., U.S. Patent 5,466,410 to Hills, and U.S. Patent 5,069,970 and U.S. Patent 5,057,368 to Largman et al., which describe hybrids with unconventional shapes. A nonwoven web of spunbond fibers produced by melt spinning is referred to as a "spunbond."

As used herein, the term "meltblown fibers" means fibers formed by extruding a molten thermoplastic material through a plurality of fine, usually circular, die capillaries as molten threads or filaments into converging high velocity, usually hot, gas (for example, air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers, Such a process is disclosed, for example, by U.S. Patent 3,849,241 to Butin et al. Meltblown fibers are microfibers which may be continuous or discontinuous, are generally smaller than 10 microns in average diameter.

As used herein, the term "bonded carded web" refers to webs made from staple fibers which are sent through a combing or carding unit, which breaks apart and aligns the staple fibers in the machine direction to form a generally machine direction-oriented fibrous nonwoven web. Such fibers are usually purchased in bales which are placed in a piker or fiberizer which separates the fibers prior to the carding unit. Once the web is formed, it is then bonded by one or more of several known bonding methods.

As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" also includes all possible geometric configurations of the molecule. These configurations include, but are not limited to, isotactic, syndiotactic and random symmetries.

As used herein, the term "microfibers" refers to small diameter fibers having an average diameter not greater than about 50 microns, for example, having an average diameter of from about 0.5 microns to about 40 microns, or more particularly, having an average diameter of from about 2 microns to about 25 microns. Another frequently used expression of fiber diameter is denier, which is defined as grams per 9000 meters of a fiber, and may be calculated as fiber diameter in microns squared, multiplied by the density in grams/cc, multiplied by 0.00707. A lower denier indicates a finer fiber and a higher denier indicates a thicker or heavier fiber. For example, a diameter of a polypropylene fiber given as 15 microns may be converted to denier by squaring, multiplying the results by 0.89 g/cc and multiplying by 0.00707. Thus, a 15 micron polypropylene fiber has a denier of about 1.42. Outside the United States, the unit of measurement is more commonly the "tex," which is defined as the grams per kilometer of fiber. Tex may be calculated as denier/9.

As used herein, the term "blend" means a mixture of two or more polymers while the term "alloy" means a sub-class of blends wherein the components are immiscible but have been compatibilized. "Miscibility" and "immiscibility" are defined as blends having negative and positive values, respectively, for the free energy of mixing. Further, "compatibilization" is defined as the process of modifying the interfacial properties of an immiscible polymer blend in order to make an alloy.

As used herein, the term "bicomponent fibers" refers to fibers which have been formed from at least two polymers extruded from separate extruders but spun together to form one fiber. Bicomponent fibers are also sometimes referred to as conjugate fibers or multicomponent fibers. The polymers are arranged in substantially constantly positioned distinct zones across the cross-sections of the bicomponent fibers and extend continuously along the length of the bicomponent fibers. The configuration of such a bicomponent fiber may be, for example, a sheath/core arrangement wherein one polymer is surrounded by another, or may be a side-by-side arrangement, a pie arrangement, or an "islands-in-the-sea" arrangement. Bicomponent fibers are taught by U.S. Patent 5,108,820 to Kaneko et al., U.S. Patent 4,795,668 to Krueger et al., U.S. Patent 5,540,992 to Marcher et al., and U.S. Patent 5,336,552 to Strack et al. Bicomponent fibers are also taught by U.S. Patent 5,382,400 to Pike et al. For two component fibers, the polymers may be present in ratios of 75/25, 50/50, 25/75 or any other desired ratio.

As used herein, "heat treating" refers to heating a material suitable for use in the method of this invention to provide the material with memory when it is necked to enable the material to return to its necked condition. In U.S. Patent 4,965,122 to Morman, a reversibly necked material having the capability of stretching at least about 75 percent and recovering at least about 50 percent when stretched about 75 percent, typically in the direction generally parallel to the direction of necking is made by applying a tensioning force to a material to neck the material, heating the necked material, and cooling the necked material.

The terms "elastic" and "elastomeric" are used interchangeably to mean a material that is generally capable of recovering its shape after deformation when the deforming force is removed. Specifically, as used herein, elastic or elastomeric is meant to be that property of any material which upon application of a biasing force, permits that material to be stretchable to a stretched biased length which is at least about 25 percent greater than its relaxed unbiased length, and that will cause the material to recover at least 40 percent of its elongation upon release of the stretching elongating force. A hypothetical example which would satisfy this definition of an elastomeric material would be a one (1) inch sample of a material which is elongatable to at least 1.25 inches (3.1 cm) and which, upon being elongated to 1.25 inches (3.1 cm) and released, will recover to a length of not more than 1.15 (2.9 cm) inches. Many elastic materials may be stretched by much more than 25 percent of their relaxed length, and many of these will recover to substantially their original relaxed length upon release of the stretching, elongating force. This latter class of materials is generally beneficial for purposes of the present invention.

As used herein, the term "personal care absorbent article" means disposable diapers, training pants, absorbent underpants, adult incontinence products, feminine hygiene products including sanitary pads and napkins, wipes, tissues, bandages, dressings and the like.

Nonwoven web materials having cross-machine direction extensibility are produced in accordance with the method of this invention by conveying a nonwoven web through means for elongating the nonwoven web in the cross-machine direction and necking the resulting cross-machine direction elongated nonwoven web, thereby forming the nonwoven web materials with cross-machine direction extensibility. Elongation of the nonwoven web in the cross-machine direction may be carried out by any means known to those skilled in the art, such as tenter frame equipment, but is preferably carried out by an intermeshing grooved roll system, various implementations of which are taught, for example, by U.S. Patent 3,383,449 to Müller, U.S. Patent 3,849,526 to Müller et al,; U.S. Patents 4,116,892; 4,153,751; 4,223,059; and 4,289,832; all to Schwarz (the BIAX process); U.S-Patent 4,350,655 to Hoge; U.S. Patent 4,517,714 to Sneed et al.; and U.S. Patent 4,806,300 to Walton et al. The amount of cross-machine direction stretch is a function of the depth to which the grooved rolls are set; the deeper the grooved rolls are meshed, the greater is the percent cross-machine direction extension. Addition of heat in the grooved rolls or during cross-machine direction orientation can further enhance orientation and reduce damage to the material. Due to the uniqueness of the cross-machine direction characteristics of different nonwoven web materials, the rolls are adjusted so as not to tear the material. In addition to stretching or elongating the nonwoven web in the cross-machine direction, the grooved roll system provides a further benefit of softening the material.

After elongation, the nonwoven web is necked, forming a cross-machine direction extensible nonwoven web material. Necking may be carried out by any method known to those skilled in the art. In accordance with one preferred embodiment of this invention, necking of the nonwoven web is achieved by applying a machine direction tension thereto. In order to provide integrity to the extensible nonwoven web material, the material may be bonded by any suitable bonding process such as through-air bonding or point bonding.

Depending upon the degree of necking, different characteristics are imparted to the CD extensible nonwoven web material. In accordance with one method, not within the scope of this invention, the elongated nonwoven web material is necked back to its original width, which corresponds to the full machine width, and then laminated to produce a CD stretchable material. As a result, the facing material of the laminate has CD extension while still taking advantage of the full width of the machine. This, in rum, enables full capacity operation of the machine, resulting in the realization of cost savings.

In accordance with another embodiment of this invention, the nonwoven web is sent through an intermeshing grooved roller system in which the cross-machine direction stretch of the nonwoven web is increased by at least about 50%. The CD stretched web is then necked or narrowed by application of machine direction tension. At this point in the process, the nonwoven web does not have much retractive force when it is stretched to its widened width. While in its necked width, the web is heat treated to lock in the CD extensibility. Heat treating of the web in this manner increases the retractive force. The amount of CD extension attainable by the nonwoven web produced in accordance with this embodiment of the method of this invention may be as high as about seven times the necked width.

More particularly, the nonwoven web is processed through a grooved roll process that widens the web from "X" inches (1 inch=2·54 cm) wide to "Y" inches wide (See Fig. 1). As the "Y" inch wide material exits the grooved roll process, it is immediately necked to about its original width "X." To increase the retractive force when the web is stretched to its widened width "Y," the web is heat-set while in its necked width "X."

In accordance with this invention, the web is further tensioned to neck the material to width "Z" inches, which is smaller than the original width "X," and then heat-set. The stretching of the nonwoven web in the cross-machine direction and then necking it to less than its original width produces a very high CD stretch nonwoven - higher than just necking by itself. For example, the grooved roll process can readily double the effective width, "X," and some webs can readily be necked to about 1/4 of their original width. The combination of the grooved rolls and the high necking with heat-treating produces a web having about 700% stretch with recovery. Referring to Fig. 1, if a nonwoven web has an original width ("X") of 10 inches (25·4 cm) and it is stretched in the cross-machine direction to a stretched width ("Y") of 20 inches (50·8 cm) the percent stretch is 100% ((Y-X)/X) x 100. If the elongated nonwoven web is then necked down to ½ of its original width to a necked width ("Z") of 5 inches (12·7 cm) and heat-set so as to retain its necked width, the percent stretch is now 300% ((Y - Z)/Z) x 100.

In accordance with one embodiment of this invention, the CD extensible nonwoven web material is bonded to an elastomeric material, producing a laminate with cloth-like feel and CD extensibility. A method for forming a composite elastic necked-bonded material in which a neckable material is necked and then joined to an elastic sheet is taught, for example, by U.S. Patent 5,226,992 to Morman. Alternatively, the CD extensible nonwoven web material may be joined to an elastic sheet in such a manner as to provide a laminate having multi-directional stretch capabilities. U.S. patent 5,116,662 to Morman teaches a method for producing a composite elastic material capable of stretching in at least two directions wherein a necked material is joined to the elastic sheet at least at three locations arranged in a nonlinear configuration such that the necked web is gathered between at least two of those locations. In accordance with another embodiment of this invention, the laminate is formed first and then conveyed through the grooved roller system. For example, conveyance of a non-stretchable SMS (spunbond-meltblown-spunbond) laminate having an elastic meltblown component through a grooved roller system produces a laminate having CD stretch/recovery and cloth-like aesthetics. Suitable elastomeric materials for use in the method and materials of this invention preferably are selected from the group consisting films, foams, spunbonds, meltblowns and arrays of filaments and netting. Films suitable for use in this invention include breathable films, that is, films that may be microporous. Such breathable films may be produced by aperturing or by stretch-thinning a film loaded with filler, such as $CaCO_3$ particles.

Nonwoven web materials suitable for use in the method of this invention are preferably selected from the group consisting ofspunbond, meltblown, spunbond-meltblown-spunbond laminates, coform, spunbond-film-spunbond laminates, bicomponent spunbond, bicomponent meltblown, biconstituent spunbond, biconstituent meltblown, bonded carded web, airlaid and combinations thereof.

The nonwoven web materials are preferably formed with polymers selected from the group including polyolefins, polyamides, polyesters, polycarbonates, polystyrenes, thermoplastic elastomers, fluoropolymers, vinyl polymers, and blends and copolymers thereof. Suitable polyolefins include, but are not limited to, polyethylene, polypropylene, polybutylene, and the like; suitable polyamides include, but are not limited to, nylon 6, nylon 6/6, nylon 10, nylon 12 and the like; and suitable polyesters include, but are not limited to, polyethylene terephthalate, polybutylene terephthalate and the like. Particularly suitable polymers for use in the present invention are polyolefins including polyethylene, for example, linear low density polyethylene, low density polyethylene, medium density polyethylene, high density polyethylene and blends thereof; polypropylene; polybutylene and copolymers as well as blends thereof. Additionally, the suitable fiber forming polymers may have thermoplastic elastomers blended therein.

Test Procedures

Cup Crush Test (Softness)

The conformability and drapeability of a nonwoven fabric may be measured according to the "cup crush" test. The cup crush test evaluates the fabric by measuring the peak load and energy required for a 4.5cm diameter hemispherically shaped foot to crush a 23cm by 23 cm piece of fabric shaped into an approximately 6.5 cm diameter by 6.5 cm tall inverted cup while the cup shaped fabric is surrounded by an approximately 6.5cm diameter cylinder to maintain a uniform deformation of the cup shaped fabric. An average of ten readings is used. The foot and the cup are aligned to avoid contact between the cup walls and the foot which could affect the reading. The cup crush load is measured while the foot is descending at a rate of about 0.25 inches per second (380 mm per minute) and is measured in grams. The cup crush energy is the total energy required to crush a sample which is the total energy from the start of the test to the peak load point, i.e., the area under the curve formed by the load in grams on one axis and the distance the foot travels on the other. Crush energy is, therefore, reported in gram-millimeters. Lower cup crush values indicate a more drapeable and comfortable laminate. A suitable device for measuring cup crush is a model FTD-G-500 load cell (500 gram range) available from the Schaevitz Company, Pennsauken, N.J.

Tensile Properties

This procedure measures the strip tensile/energy and elongation of a specimen. Samples are measured in the machine direction (MD) and the cross direction (CD). A sample of 3 inches (7·62 cm) x 6 inches (15·24 cm) is placed on the pneumatic jaws of an Instron tensile tester with a load cell of 10 pounds (4·54 kg) setting up the gage length to 3 inches (7·62 cm) and a crosshead speed of 12 inches (30·48 cm) minute. The sample is placed on the clamps and the equipment is started. The top clamp is lifted by the equipment at the cross head speed until the specimen breaks. The strip tensile peak load (pounds), the maximum load before the specimen ruptures, and the elongation at break (%) (peak strain) are read from the instrument. The modulus is calculated in the typical manner as the slope of the best fitting line on a stress/strain curve as calculated from zero to the proportional limit. The energy is calculated with the hollowing formula:

$$E = R/500 \times L \times S$$

where

E = Energy (inch per pound)
R = Integrator reading
L = Full scale load in pounds
S = Crosshead speed (inch/minute)

This is performed at a constant temperature of 73 +/- 2°F and a relative humidity of 50 +/-2%.

Examples

A web of bicomponent fibers comprising one-half polyethylene and one-half polypropylene in a side-by-side configuration, which, upon heating, results in the softening of the lower melting point polyethylene, thereby enabling the fibers to fuse together, was sent through a BIAX grooved roll process to produce a 100% CD extensible web. The web was then stretched in a Sintec Tensile Tester to neck the material to about ½ of its original width. When the tension was released, the material relaxed to about its original width. The test was then repeated except that the material while in the necked condition was heated, as determined by a Raytek Raynger Model STAL infrared gun (available from Raytek Corporation, Santa Cruz, CA), to a temperature in the range of about 150°F to about 170°F using a Revlon 1800 watt hair dryer for about 45 seconds. When the tension was released, the material did not relax to its original width, but rather the material remained approximately at its necked width. As a result, the retractive properties of the material were greatly increased.

The following examples show the effect of processing nonwoven materials through a grooved roll system and result in cross direction extension after "necking"of the material in accordance with the method of this invention.

To simulate the "necking" process, the following samples were prepared from bicomponent polyethylene/polypropylene prism (a bicomponent fiber produced in accordance with the method of, for example, U.S. Patent 5,382,400, wherein two different polymers are extruded through the same extruder opening) spunbond having a basis weight of about 25 gsm and KRATON (Shell Chemical Company, Houston, TX) meltblown (MB) material as described in U.S. Patent 4,663,220 having a basis weight of about 144 gsm:

1. Spunbond control
2. Spunbond control plus KRATON MB
3. Grooved spunbond plus KRATON MB
4. Grooved spunbond necked from 6 inches (15 cm) to 4.25 inches (10.6 cm) plus KRATON MB
5. Grooved spunbond necked from 6 inches (15 cm) to 3 inches (7.5 cm) plus KRATON MB
6. Grooved spunbond necked from 6 inches (15 cm) to 3 inches (7.5 cm) plus MD stretched KRATON MB (final laminate width expanded to 4 inches (10 cm))
7. Spunbond control necked from 6 inches (15, cm) to 3 inches (7.5 cm) plus KRATON MB

Table 1 hereinbelow compares the difference between the control spunbond and the control spunbond with KRATON meltblown MB. The data show that when the spunbond is attached to the KRATON meltblown, all measured tensile properties are increased.

| Table 1: Cross-Direction Tensile Properties of Sample 1 vs. Sample 2 | | | |
|---|---|---|---|
| Sample | Basis Weight (gsm) | Peak Load (gm) | Peak Strain (%) |
| 1 | 25 | 866.2 | 65 |
| 2 | 177 | 1401.5 | 96.5 |

Table 2 compares the control spunbond/KRATON MB sample with the grooved spunbond/KRATON MB. The control spunbond/KRATON MB had higher tensile peak load than the grooved spunbond/KRATON MB laminate. In measuring final CD extension, the grooved spunbond/KRATON MB material had higher peak strain percentages compared to the control spunbond/KRATON MB material.

| Table 2: Cross-Direction Tensile Properties of Sample 2 vs. Sample 3 | | | |
|---|---|---|---|
| Sample | Basis Weight (gsm) | Peak Load (gm) | Peak Strain (%) |
| 2 | 177 | 1401.5 | 96.5 |
| 3 | 165 | 883.6 | 174.8 |

Table 3 sets forth the differences between Samples 3 and 4 where Sample 4 consisted of a grooved spunbond that was necked from 6 inches (15 cm) to 4.25 inches (10.6 cm) to approximately match the original basis weight of the spunbond prior to passing through the grooved rolls. As can be seen, peak strain increased slightly.

| Table 3: Cross-Direction Tensile Properties of Sample 3 vs. Sample 4 | | | |
|---|---|---|---|
| Sample | Basis Weight (gsm) | Peak Load (gm) | Peak Strain (%) |
| 3 | 165 | 883.6 | 174.8 |
| 4 | 220 | 1122.1 | 189.9 |

Table 4 hereinbelow sets forth the .difference between a grooved spunbond/KRATON MB material and a necked grooved spunbond/KRATON MB material. As shown, peak strain increased with necking.

| Table 4: Cross-Direction Tensile Properties of Sample 3 vs. Sample 5 | | | |
|---|---|---|---|
| Sample | Basis Weight (gsm) | Peak Load (gm) | Peak Strain (%) |
| 3 | 165 | 883.6 | 174.8 |
| 5 | Not measured | 1300 | 388.7 |

Tables 5a and 5b compare the material of Samples 6 and 7. Table 5a shows the properties in cross-direction and Table 5b shows the properties in machine direction. Sample 6 was made to be biaxially extensible. The grooved spunbond was necked and adhesively laminated to a KRATON MB material that was stretched in the machine direction. Both the spunbond material and the KRATON MB material were under tension during lamination. Sample 7 was made to provide cross-direction extensibility. The control spunbond material was MD stretched and 50% "neck in" was achieved. The KRATON MB material was adhesively laminated to the control spunbond. The results show that Sample 6 had high MD extensibility but somewhat lower CD extensibility when compared to Sample 7.

| Table 5a: Cross-Direction Tensile Properties of Sample 6 and Sample 7 | | | |
|---|---|---|---|
| Sample | Basis Weight (gsm) | Peak Load (gm) | Peak Strain (%) |
| 6 | 118 | 1460.0 | 191.2 |
| 7 | 127 | 1852.5 | 263 |

| Table 5b: Machine-Direction Tensile Properties of Sample 6 and Sample 7 | | | |
|---|---|---|---|
| Sample | Basis Weight (gsm) | Peak Load (gm) | Peak Strain (%) |
| 6 | 118 | 2858.5 | 182.1 |
| *7 | 127 | 5098 | 75.8 |
| *Only one repeat due to limited sample size | | | |

Table 6 shows a comparison of a grooved spunbond/KRATON (3), a grooved spunbond necked from 6 inches (15 cm) to 3 inches (7.5 cm) plus KRATON (5), and a control spunbond necked from 6 inches (15 cm) to 3 inches (7.5) plus KRATON (7) material. Of all three samples, the necked grooved spunbond/KRATON MB material provided the highest peak strain percent. This indicates that the necked grooved spunbond/KRATON (5) sample is more extensible compared to Samples 3 and 7.

| Table 6: Cross-Direction Tensile Properties of Sample 2, Sample 5 and Sample 7 | | | |
|---|---|---|---|
| Sample | Basis Weight (gsm) | Peak Load (gm) | Peak Strain (%) |
| 3 | 165 | 883.6 | 174.8 |
| 5 | Not measured | 1300 | 388.7 |
| 7 | 127 | 1852.5 | 263 |

A spunbond-meltblown-spunbond (SMS) hand sample was passed through the cross direction rollers of a grooved roll system. The meltblown layer of the SMS was composed of metallocene elastomeric polyolefin (Dow ENGAGE, 30 melt index, 0.5 osy, available from Dow Chemical Company). A 20 percent CD orientation was achieved with 0.150 inches (0.375 cm) engagement. Cross direction (CD) percent orientation is a measured quantity determined by putting a circle on a material, feeding the material through a grooved roller process, and measuring the resulting circle at its widest diameter. The percentage increase is the calculated value ((final diameter - initial diameter)/initial diameter) x 100. The material is fed through the grooved roll process as a hand sheet so there is minimal tension going into the grooved rolls and no tension coming out from between the grooved rolls. The production of materials having cross direction percent orientation is taught, for example, by U.S. Patent 4,368,565.

Table 7 compares the control spunbond-meltblown (elastomeric)-spunbond (SMS) and grooved oriented SMS samples. The results show that grooved SMS sample had a lower tensile peak load but a higher peak strain.

| Table 7: Cross-Direction Tensile Properties of Sample A vs. Sample B | | | |
|---|---|---|---|
| Sample | Basis Weight (gsm) | Peak Load (gm) | Peak Strain (%) |
| A | 97 | 3868 | 66.9 |
| B | 80 | 976.5 | 118.6 |
| A. SMS @ 97 GSM<br>B. Grooved SMS @ 80 GSM | | | |

A hand sample of stretched-bonded laminate (SBL) material was passed through BIAX CD rollers. A 14% final CD orientation was achieved with 2 passes at 0.10 inches (0.25 cm) engagement. This material was biaxially elastic.

The data in Table 8 show the differences between a spunbond-meltblown (elastomeric)-spunbond material, a grooved spunbond-meltblown (elastomeric)-spunbond material, a necked-bonded-laminate (NBL) material, and a stretched-bonded-laminate (SBL) material. When the SMS sample was subjected to grooved roll stretching, the result was a softer laminate as indicated by the cup crush data. The grooved roll SMS sample also had a higher air permeability compared to the control SMS sample. The NBL sample consists of spunbond and film and, thus, is not air permeable. In comparison with the SBL sample, the grooved SMS sample had a higher air permeability in both relaxed and CD stretched state.

| Table 8: Air Permeability and Softness Properties of SMS and Grooved SMS | | | | | |
|---|---|---|---|---|---|
| Sample | Basis Weight (gsm) | Relaxed State Air Permeability (cfm) | CD Stretched Air Permeability (cfm) | Cup Crush Load (gms) | Cup Crush Energy (gm/mm) |
| SMS | 97 | 274 | Can't stretch | 173 | 3422 |
| Grooved SMS | 80 | 446 | 568 | 38 | 459 |
| NBL | 96 | 0 | 0 | Did not measure | Did not measure |
| SBL | 133 | 239 | 339 | Did not measure | Did not measure |

While in the foregoing specification this invention has been described in relation to certain preferred embodiments thereof, and many details have been set forth for purpose of illustration, it will be apparent to those skilled in the art that the invention is susceptible to additional embodiments and that certain of the details described herein can be varied considerably without departing from the basic principles of the invention.

Claims

1. A method for producing a nonwoven web material having cross-machine direction extensibility comprising the steps of:

conveying a nonwoven web having an original width (X) through means for elongating said nonwoven web in a cross-machine direction (Y); and
then necking said elongated nonwoven web to a width (Z) less than said original width (X), forming a cross-machine direction extensible nonwoven web material;

**characterized in that** said method further comprises the step of:

> heating said necked elongated nonwoven web to a temperature suitable for heat-treating said necked elongated nonwoven web.

2. A method in accordance with Claim 1, wherein said nonwoven web is necked by application of machine direction tension.

3. A method in accordance with Claim 1 or 2, wherein said extensible nonwoven web is attached to an elastomeric material, forming a cross-machine direction stretchable laminate.

4. A method in accordance with Claim 3, wherein said elastomeric material is a material selected from the group consisting of a film, a foam, a meltblown material, a spunbond material, an array of filaments and netting, and laminates thereof.

5. A method in accordance with Claim 1 or 2, wherein said extensible nonwoven web is attached to an elastomeric material, forming a multi-direction stretchable laminate.

6. A method in accordance with any preceding claim, wherein said nonwoven web comprises material selected from the group consisting of spunbond, meltblown, airlaid, coform, bonded carded web and laminates thereof.

7. A method in accordance with any of Claims 1 to 5, wherein said nonwoven web is a spunbond-meltblown-spunbond laminate having an elastomeric meltblown component.

8. A method in accordance with any preceding claim, wherein said nonwoven web is elongated in said cross-machine direction by at least about 50%.

9. A method in accordance with any preceding claim, wherein said extensible nonwoven web material is highly stretchable.

**Patentansprüche**

1. Verfahren zum Herstellen eines Vliesbahnmaterials mit einer Dehnbarkeit in Quermaschinenrichtung, welches die Schritte umfasst:

> Befördern einer Vliesbahn mir einer Originalbreite (X) durch Mittel zum Verlängern der Vliesbahn in einer Quermaschinenrichtung (Y); und
> dann Verengen der verlängerten Vliesbahn auf eine Breite (Z), die geringer ist als die Originalbreite (X), wobei eine in Quermaschinenrichtung dehnbare Vliesbahn gebildet wird;

> **dadurch gekennzeichnet, dass** das Verfahren des Weiteren den Schritt umfasst:

> Erwärmen der verengten verlängerten Vliesbahn auf eine Temperatur, die zur Wärmebehandlung der verengten verlängerten Vliesbahn geeignet ist.

2. Verfahren gemäß Anspruch 1, wobei die Vliesbahn durch Anwenden einer Maschinenrichtungsspannung verengt wird.

3. Verfahren gemäß Anspruch 1 oder 2, wobei die dehnbare Vliesbahn an einem elastomeren Material befestigt ist, wobei ein in Quermaschinenrichtung dehnbares Laminat gebildet wird.

4. Verfahren gemäß Anspruch 3, wobei das elastomere Material ein Material ist, welches ausgewählt ist aus der Gruppe bestehend aus einem Film, einem Meltblown-Material, einem Spunbond-Material, einem Feld aus Filamenten und Verknüpfungen und aus Laminaten davon.

5. Verfahren gemäß Anspruch 1 oder 2, wobei die dehnbare Vliesbahn an einem elastomeren Material befestigt ist, wobei ein in vielen Richtungen dehnbares Laminat gebildet wird.

**6.** Verfahren gemäß einem der vorherigen Ansprüche, wobei die Vliesbahn ein material umfasst, welches ausgewählt ist aus der Gruppe bestehend aus Spunbond, Meltblown, Airlaid, Coform, gebunden kardierter Bahn und aus Laminaten davon.

**7.** Verfahren gemäß einem der Ansprüche 1 bis 5, wobei die Vliesbahn ein Spunbond-Meltblown-Spunbond-Laminat mit einer elastomeren Meltblown-Komponente ist.

**8.** Verfahren gemäß einem der vorherigen Ansprüche, wobei die Vliesbahn in der Quermaschinenrichtung um mindestens ungefähr 50% verlängert wird.

**9.** Verfahren gemäß einem der vorherigen Ansprüche, wobei das dehnbare Vliesbahnmaterial stark dehnbar ist.


**Revendications**

**1.** Procédé pour produire un matériau de voile non tissé ayant une extensibilité dans un sens travers, comprenant les étapes de ;
transport d'un voile non tissé ayant une largeur d'origine (X) à travers des moyens pour allonger ledit voile non tissé dans un sens travers (Y) ; et
étranglement ensuite dudit voile non tissé allongé jusqu'à une largeur (Z) inférieure à ladite largeur d'origine (X), en formant un matériau de voile non tissé extensible dans le sens travers ;
**caractérisé en ce que** ledit procédé comprend en outre l'étape de :

chauffage dudit voile non tissé allongé étranglé jusqu'à une température adaptée pour traiter thermiquement ledit voile non-tissé allongé étranglé.

**2.** Procédé selon la revendication 1, dans lequel ledit voile non-tissé est étranglé par application d'une tension dans le sens machine.

**3.** Procédé selon la revendication 1 ou 2, dans lequel ledit voile non-tissé extensible est attaché à un matériau élastomère, en formant un stratifié étirable dans le sens travers.

**4.** Procédé selon la revendication 3, dans lequel ledit matériau élastomère est un matériau choisi parmi le groupe constitué d'un film, d'une mousse, d'un matériau extrudé-soufflé, d'un matériau filé-lié, d'un réseau de filaments et d'un filet, et de stratifiés de ceux-ci.

**5.** Procédé selon la revendication 1 ou 2, dans lequel ledit voile non-tissé extensible est attaché à un matériau élastomère, en formant un stratifié étirable dans des directions multiples.

**6.** Procédé selon l'une quelconque des revendications précédentes, dans lequel ledit voile non-tissé comprend un matériau choisi parmi le groupe constitué d'un voile filé-lié, d'un voile extrudé-soufflé, d'un voile appliqué avec de l'air, d'une coforme, d'un voile cardé lié et de stratifiés de ceux-ci.

**7.** Procédé selon l'une quelconque des revendications 1 à 5, dans lequel ledit voile non-tissé est un stratifié filé-lié/extrudé-soufflé/filé-lié ayant un composant élastomère extrudé-soufflé.

**8.** Procédé selon l'une quelconque des revendications précédentes, dans lequel ledit voile non-tissé est allongé dans ledit sens travers d'au moins environ 50 %.

**9.** Procédé selon l'une quelconque des revendications précédentes, dans lequel ledit matériau de voile non-tissé extensible est fortement étirable.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4720415 A, Vander Wielen
- US 4965122 A, Morman
- US 5914084 A
- US 4340563 A, Appel
- US 3692618 A, Dorschner
- US 3802817 A, Matsuki
- US 3338992 A
- US 3341394 A, Kinney
- US 3502763 A, Hartmann
- US 3542615 A, Dobo
- US 5277976 A, Hogle
- US 5466410 A, Hills
- US 5069970 A
- US 5057368 A, Largman
- US 3849241 A, Butin
- US 5108820 A, Kaneko
- US 4795668 A, Krueger
- US 5540992 A, Marcher
- US 5336552 A, Strack
- US 5382400 A, Pike
- US 3383449 A, Müller
- US 3849526 A, Müller
- US 4116892 A
- US 4153751 A
- US 4223059 A
- US 4289832 A, Schwarz
- US 4350655 A, Hoge
- US 4517714 A, Sneed
- US 4806300 A, Walton
- US 5226992 A, Morman
- US 5116662 A, Morman
- US 4663220 A
- US 4368565 A